# FASCICULE DE BREVET EUROPEEN

(11) **EP 0 953 343 B1**
(45) Date de publication et mention de la délivrance du brevet: **31.03.2004**
(21) Numéro de dépôt: 99400733.4
(22) Date de dépôt: 25.03.1999
(51) Int. Cl.: A61K 7/48, A61K 7/42

(54) **Composition cosmétique contenant un nouveau pigment**
Kosmetische Zusammensetzung enthaltend ein neues Pigment
Cosmetic composition containing a new pigment

(30) Priorité: 27.03.1998 FR 9803839
(43) Date de publication de la demande: 03.11.1999
(73) Titulaire: L'OREAL, 75008 Paris (FR)
(72) Inventeur: Lemann, Patricia, 94000 Créteil (FR); Simon, Jean-Christophe, 75012 Paris (FR)
(74) Mandataire: Lhoste, Catherine

(56) Documents cités:
- EP-A- 0 719 842
- EP-A- 0 787 730
- EP-A- 0 787 731
- EP-A- 0 811 625

## Description

La présente invention se rapporte à des compositions cosmétiques contenant un nouveau pigment rouge de couleur intense et saturée, non générateur de radicaux libres et plus spécialement à des compositions de maquillage de la peau aussi bien du visage que du corps humain, des fibres kératiniques ou phanères comme les ongles, les cils, les sourcils ou les cheveux et des lèvres.

Les compositions de maquillage telles que les poudres libres ou compactées, les fonds de teint, les fards à joues ou à paupières, les rouges à lèvres, les anti-cernes, les blushes, les mascaras, les eye-liners, les crayons à lèvres ou à yeux ou encore les vernis à ongles et les produits de maquillage du corps, sont constituées d'un véhicule approprié et d'agents de coloration de natures différentes, destinés à conférer une certaine couleur à ces compositions, avant et/ou après leur application sur la peau, les lèvres et/ou les phanères.

Ces agents de coloration peuvent être des laques, des pigments minéraux ou organiques et/ou des pigments nacrés ou encore des colorants. Dans la gamme des pigments rouges, les cosméticiens disposent de pigments d'origine minérale comme les oxydes de fer rouges ou les mélanges d'oxydes de fer brun-jaune et de pigments d'origine organique. Les pigments minéraux, et en particulier les oxydes minéraux ont l'avantage d'être très stables mais ont l'inconvénient de donner des couleurs plutôt ternes et pâles. Les laques organiques ont l'avantage de conférer aux compositions des couleurs vives, mais sont pour la plupart instables à la lumière, à la température ou au pH. Certaines de ces laques présentent également l'inconvénient de tacher la peau de manière disgracieuse après application, par dégorgement du colorant. Les pigments nacrés quant à eux permettent d'obtenir des couleurs variées, mais jamais intenses, à effets irisés mais le plus souvent assez faibles.

Par ailleurs, certains agents de coloration présentent l'inconvénient d'engendrer des radicaux libres dans les formules de maquillage modifiant le rendu des couleurs et la stabilité des compositions, puis sur la peau après application, ce qui favorise le vieillissement cutané (apparition de rides, ridules, jaunissement de la peau). Comme agents de coloration présentant cet inconvénient ont peut citer notamment les mélanges d'oxydes de fer brun-jaune vendu sous la dénomination commerciale « Sicomet Brun ZP 3569 » par BASF par exemple, les pigments d'origine organique comme le Rouge Flaming ( D&C Red n°36, Color Index 12085) vendu sous la dénomination commerciale D&C Red 36W 008 par WACKER ainsi que la laque d'aluminium de phloxine B sur alumine (D&C Red n° 27 Aluminium Lake).

Aujourd'hui pour pallier cet inconvénient, on utilise des agents anti-oxydants comme par exemple l'éthoxyquine. Malheureusement, il est souvent difficile de trouver un agent anti-oxydant efficace à 100% compte tenu de la multiplicité des ingrédients présents dans les compositions de maquillage. De plus, les agents anti-oxydants engendrent souvent eux-mêmes des produits de dégradation (oxydation de l'agent anti-oxydant) qui peuvent être gênants.

La présente invention a justement pour objet l'utilisation dans une composition cosmétique d'un nouveau pigment rouge de couleur intense et saturée, stable ayant l'avantage d'engendrer beaucoup moins de radicaux libres que les pigments classiquement utilisés notamment pour obtenir une couleur rouge.

De façon surprenante, le demandeur a trouvé que les dicéto pyrrolo-pyrroles (DPP en abrégé) permettaient de limiter la production de radicaux libres, puisqu'ils ont la propriété d'engendrer très peu de radicaux libres, et d'éviter ainsi l'utilisation d'anti-oxydants dans les compositions. En outre ces pigments permettent d'obtenir une coloration intense et en particulier un rouge vermillon, très vif et intense, non dégorgeante sur la peau, stable à la lumière, au pH et à la température.

On connaît par EP0811625 un procédé de préparation de dérivés de dicétopyrrolopyrroles; on connaît également par EP0719842 des compositions comprenant un pigment de petite taille et un agent 'flop-enhancing'; on connaît encore par EP0787730 et EP0787731, des dicétopyrrolopyrroles polymérisables ainsi que les polymères les comprenant.

De façon plus précise, l'invention a pour objet une composition cosmétique à application topique colorée et plus spécialement une composition cosmétique de maquillage caractérisée par le fait qu'elle comprend 0,01 à 50% en poids par rapport au poids de la composition, un pigment de formule (**A**) : 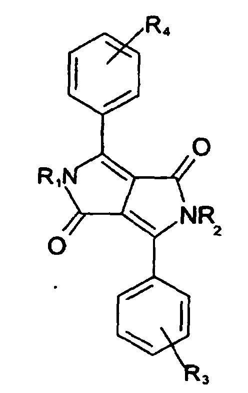 dans laquelle R₁ et R₂ représentent indépendamment un atome d'hydrogène, un radical alkyle en C₁ à C₁₈, alcényle en C₃ à C₁₂, alkoxycarbonyle en C₂ à C₅, phényle éventuellement substitué par un halogène, R₃ et R₄ représentent indépendamment un atome d'hydrogène, un groupement SO₃M, un radical alkyle en C₁ à C₁₈, alkoxy en C₁ à C₄, halogène, phényle, les radicaux alkyle, alcényle et alkoxy pouvant être linéaires ou ramifiés, M représentant un atome d'hydrogène, un atome de métal, par exemple de sodium, potassium ou lithium, ou un groupement ammonium. Les radicaux R₃ peuvent être en position 2, 3, 4, 5 ou 6 et de préférence en position 4.

Les radicaux alkyle et alkoxy peuvent être linéaires ou ramifiés et être choisis notamment parmi les radicaux méthyle, éthyle, n- et iso-propyle, n-, sec-, tert- ou iso-butyle, n-, sec-, tert- ou iso-pentyle, etc. ; les radicaux alcényle peuvent être linéaires ou ramifiés et être choisis notamment parmi les radicaux allyle, méthallyle, 2-butényle, 2-hexényle, 3-hexényle, 2-octényle, etc. L'atome d'halogène peut être CI ou F. De préférence, R₁ et R₂ représentent indépendamment un atome d'hydrogène, un radical méthyle ou 4-chlorophényle. Avantageusement, R₃ et R₄ représentent indépendamment un atome d'hydrogène, 4-chloro ou 4-ter-butyle.

Le pigment objet de l'invention peut se présenter sous forme transparente ou opaque et être associé à d'autres pigments pour augmenter la stabilité du pigment DPP et de la composition à la lumière et aux intempéries, comme décrit dans le document WO 98/56859 ou pour augmenter les propriétés colorantes et/ou les propriétés goniochromatiques de pigments à cristaux liquides ou multicouches ayant des propriétés goniochromatiques.

La fabrication des dicéto diaryl pyrrolo-pyrroles de formule (**A**) est décrite notamment dans les documents de Ciba -Geigy EP-A-542669, EP-A-787730, EP-A-787731 et WO-A-96/08537.

De préférence, on utilise le « Rouge Irgazine DPP-red BO » répertorié color index (CI en abrégé) Pigment Red 254 - N° CI 56110. Il s'agit du pyrrolo (3,4 -c) pyrrole-1,4-dione dont les deux cycles benzéniques sont dihydrogénés en 2, 5 et chlorés en 4. Ce composé présente la formule (**B**) suivante :

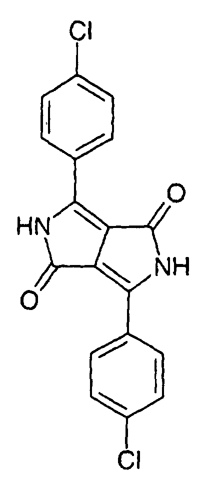

Comme autre pigment « DPP » utilisable dans la composition de l'invention, on peut citer :
- Cromophtal DPP Red BP P - Cl Pigment Red 254 - N° CI 561 10 ;
- Cromophtal DPP Red BP - CI Pigment Red 254 - N° CI 56110 ;
- Irgazine DPP Red 5G - Cl Pigment Red 255 ;
- Irgazine DPP Rubine TR - Cl Pigment Red 264 ;
- Irgazine DPP Red 5049 B - Cl Pigment Red 270 ;
- Irgazine Orange RA - Cl Pigment Orange 73.

Pour montrer la propriété qu'a le DPP de ne pas engendrer de radicaux libres, le demandeur a réalisé un test d'éthylène selon le procédé décrit dans l'article « Ethylene formation from methionine as a method to evaluate oxygen free radical scavenging and metal inactivation by cosmetics » de J.-B. Galey, F. Millecamps et Q.-L. Nguyen, *International Journal of Cosmetic Science*, 13, 65-78, 1991.

L'objectif est de comparer le comportement du pigment organique selon l'invention, par rapport à celui de pigments classiques dans un test de photo-oxydation utilisant le fer comme générateur de radicaux libres.

Dans le protocole de mesure du test d'éthylène, le FeCl₃ utilisé pour activer la production de radicaux libres a été substitué par chacun des pigments à tester. Les résultats sont donnés dans le tableau ci-après.

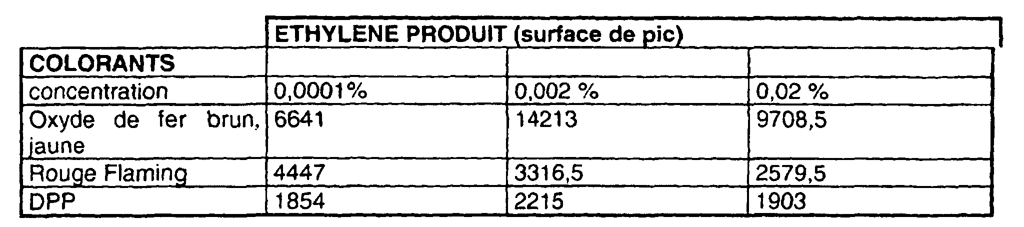

Le témoin FeCl₃ à 0,005 % est en moyenne à 9000 (unité arbitraire - mesure relative).

Plus la quantité d'éthylène est élevée, plus la production de radicaux libres est élevée.

Les matières premières oxyde de fer brun, jaune (CI 77491 + CI 77492) et rouge Flaming (CI 12085), ne sont pas inertes. A faible dose, elles activent jusqu'à une certaine concentration où l'effet protecteur du pigment commence à jouer, tandis que pour le DPP, le taux d'éthylène produit est très faible par rapport aux deux autres et n'évolue pas en fonction de la concentration. Ce pigment pourra donc être avantageusement utilisé dans des .compositions de maquillage et des compositions solaires notamment colorées destinées à la protection de la peau et/des muqueuses telles que les lèvres, sans engendrer de radicaux libres et donc limiter ainsi la dégradation de la peau et/ou des muqueuses.

Le pigment selon de l'invention peut être incorporé dans une composition cosmétique notamment de maquillage en une quantité déterminable aisément par l'homme du métier sur la base de ses connaissances générales, et qui peut notamment aller de 0,01 à 50% en poids par rapport au poids de la composition, de préférence en une quantité allant de 0,5 à 25% en poids. Le pigment peut éventuellement être fixé sur un polymère et notamment greffé ou encore être enrobé.

La composition de l'invention peut se présenter sous forme d'un produit à appliquer sur les lèvres, les yeux, la peau et/ou les phanères. Elle contient donc un milieu cosmétiquement acceptable, c'est-à-dire un milieu compatible avec toutes les matières kératiniques telles que la peau aussi bien du corps humain que du visage, les ongles, les cheveux, les cils et sourcils. Ce milieu peut comprendre ou se présenter notamment sous forme de suspension, dispersion, solution en milieu solvant, aqueux ou hydroalcoolique, éventuellement épaissie voire gélifiée ; émulsion huile-dans-eau, eau-dans-huile, ou multiple ; gel ou mousse ; gel émulsionné ; dispersion de vésicules notamment de lipides ioniques ou non ; lotion biphase ou multiphase ; spray ; poudre libre, compactée ou coulée ; pâte anhydre. L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Lorsque la composition selon l'invention se présente sous la forme d'une émulsion, elle peut éventuellement comprendre, en outre, un tensioactif, de préférence en une quantité de 0,01 à 30% en poids par rapport au poids total de la composition.

Selon l'application envisagée, la composition peut comprendre, en outre, un polymère filmogène (comme nitrocellulose, résine hydrocarbonée et/ou siliconée). Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable et éventuellement associés à des agents de coalescence et/ou des plastifiants.

La composition selon l'invention peut également comprendre une phase grasse, notamment constituée de corps gras liquides à température ambiante (25°C en général) et/ou de corps gras solides à température ambiante tels que les cires, les corps gras pâteux, les gommes et leurs mélanges.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, palmitate d'éthyl-2hexyle, stéarate d'octyl-2-dodécyle, érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol , comme le dioctanoate de propylène glycol, le diheptanoate de néopentylglycol, le diisononanoate de diéthylèneglycol et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polydiméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent représenter de 0 à 100 % en poids par rapport au poids total dé la phase grasse.

La composition selon l'invention peut, de plus, comprendre tous les ingrédients classiquement utilisés dans les domaines concernés et plus spécialement dans les domaines cosmétique et dermatologique. Ces ingrédients sont en particulier choisis parmi les conservateurs, les épaississants de phase aqueuse (biopolymères polysaccharidiques, les polymères synthétiques) ou grasse, les parfums, les actifs hydrophiles ou lipophiles et leurs mélanges. Les quantités de ces différents ingrédients sont celles classiquement utilisées dans les domaines concernés et par exemple de 0,01 % à 20 % du poids total de la composition. La nature de ces ingrédients et leur proportion doit être compatibles avec l'obtention de compositions selon l'invention, stables épaissies et brillantes. La composition peut aussi contenir de l'eau à une concentration allant de 0 à 98% du poids total de la composition.

La composition de l'invention peut, en outre, comprendre une phase particulaire additionnelle pouvant être présente à raison de 0 à 35 % du poids total de la composition, de préférence de 0,05 à 20 %, et qui peut comprendre des pigments et/ou des nacres et/ou des charges utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments autres que le DPP peuvent être présents dans la composition à raison de 0 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le stéarate de zinc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

La composition de l'invention peut comprendre, avantageusement une phase grasse solide ou pâteuse, contenant une ou plusieurs gommes et/ ou une ou plusieurs cires. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.

Les gommes sont généralement des PDMS à haut poids moléculaire et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 5 à 30 %.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition et peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des monoalcools inférieurs linéaires ou ramifiés ayant de 1 à 8 atomes de carbone, des polyols, les mono- ou di-alkyle d'isosorbide dont les groupements alkyle ont de 1 à 5 atomes de carbone, les éthers de glycol, les esters gras.

Cette composition peut avoir l'aspect d'une poudre, crème, pommade, lotion fluide, pâte souple de viscosité dynamique à 25°C de l'ordre de 1 à 40 Pa.s, onguent, solide coulé ou moulé et notamment en stick ou en coupelle.

La composition selon l'invention peut être avantageusement utilisée pour le maquillage de la peau et/ou des lèvres et/ou des phanères selon la nature des constituants utilisés. En particulier, la composition de l'invention peut être un bâton de rouge à lèvres, un brillant à lèvres (gloss en terminologie anglo-saxonne) utilisable tel quel ou pour appliquer sur un film de rouge à lèvres notamment pour en augmenter sa brillance et/ou couleur (top coat en terminologie anglo-saxonne). Elle peut aussi constituer un fond de teint solide, un produit anti-cernes ou contours des yeux, un eye liner, un mascara, une ombre à paupières, une poudre, un blush. Ces compositions peuvent, en outre, contenir des actifs cosmétiques ou dermatologiques, en vue, notamment d'apporter un aspect soin ou traitant à la composition. Ainsi la composition peut contenir des vitamines et autres actifs lipophiles (lanoline, filtre UVA) ou hydrophiles (hydratants comme la glycérine).

L'invention a encore pour objet une utilisation cosmétique de la composition ci-dessus pour soigner et/ou maquiller et/ou protéger la peau et/ou les lèvres et/ou les fibres kératiniques ainsi qu'une utilisation de cette composition pour la préparation d'un onguent destiné à traiter et/ou protéger la peau et/ou les lèvres. L'invention a aussi pour objet un procédé de traitement cosmétique de la peau et/ou des lèvres et/ou des fibres kératiniques, consistant à appliquer sur la peau et/ou les lèvres et/ou les fibres kératiniques la composition définie ci-dessus.

De façon plus spécifique, l'invention a pour objet un produit à lèvres ou un blush.

La composition de l'invention peut être obtenue par chauffage des différents constituants à la température de fusion des cires les plus élevées, puis coulage du mélange fondu dans un moule (coupelle ou doigt de gant). Elle peut aussi être obtenue par extrusion comme décrit dans la demande EP-A-667 146.

L'invention a encore pour objet l'utilisation, dans une composition cosmétique colorée d'un agent de coloration tel que décrit ci-dessus, afin de protéger la peau et/ou les lèvres et/ou les fibres kératiniques contre les méfaits des radicaux libres et/ou lutter contre les signes cutanés du vieillissement notamment photoinduit. Ces signes du vieillissement sont en particulier les rides, ridules, la peau flasque et/ou jaunie.

L'invention a encore pour objet un procédé de protection cosmétique de la peau et/ou des lèvres et/ou des fibres kératiniques, contre les méfaits des radicaux libres et/ou de lutte contre les signes cutanés du vieillissement photoinduit, consistant à appliquer sur la peau et/ou les lèvres et/ou les fibres kératiniques la composition telle que définie ci-dessus.

Les exemples de compositions ci-après sont donnés à titre illustratif et sans caractère limitatif.

### Exemple 1 : Rouge-à-lèvres :

| | |
|---|---|
| Cire de Polyéthylène | 14 g |
| Huile de Sésame | 78 g |
| Pigment DPP (formule B) | 5 g |
| Dioxyde de titane | 3 g |

### Mode opératoire :

- Homogénéisation du mélange huile + pigments pendant 45 minutes dans un bain d'huile,
- 3 passages successifs à un broyeur tricylindre,
- Homogénéisation du mélange huile + pigments pendant 30 minutes dans un bain d'huile,
- Moulage dans un moule à 42°C et 30 minutes au congélateur.

On obtient un rouge-à-lèvres d'un rouge vermillon intense, de couvrance élevée, brillant, stable à la lumière, ne laissant aucune griffe (ou marque) après démaquillage.

### Exemple 2: Blush :

| | |
|---|---|
| Talc | 38 g |
| Mica | 20 g |
| Oxychlorure de Bismuth | 8 g |
| Stéarate de zinc | 3g |
| Poudre de nylon | 20 g |
| Pigment DPP (formule B) | 5 g |
| Liant gras (*) | qsp100g |

| | |
|---|---|
| (*) Mélange d'huiles carbonées contenant : - 3,6 g de triglycérides d'acide caprique/caprylique, - 2,0 g d'isoparaffine hydrogénée (non volatile), - 1,0 g d'huile de jojoba. | |

### Mode opératoire :

- Prémélange de toutes les charges et pigments,
- 5 minutes au Lödige (mélangeur homogénéisateur de poudres),
- Addition du liant organique,
- 5 minutes au Lödige,
- Passage au jet d'air (CHRISPRO),
- Tamisage à 160 microns.

### Exemple 3 : Laque à Lèvres :

| | |
|---|---|
| Dispersion aqueuse de polymère acrylique/styrène | 20% matière active |
| (Néocryl A-1052 de Zeneca) | |
| Acétyltributylcitrate | 2,5 % |
| Pigment DPP | 1,5 % |
| Rouge Flaming (CI 12085) | 1,5 % |
| Glycérine | 1,25 % |
| Eau | qsp 100 % |

### Mode opératoire :

On ajoute à température ambiante l'acétyltributylcitrate, les pigments ; la phase aqueuse (glycérine + eau) à la dispersion de polymère puis on homogénéise.

On obtient une laque à lèvres de couleur rouge, stable et couvrante.

## Revendications

1. Composition cosmétique à application topique colorée, **caractérisée par le fait qu'**elle comprend, 0,01 à 50% en poids par rapport au poids de la composition, de pigment de formule (A) : 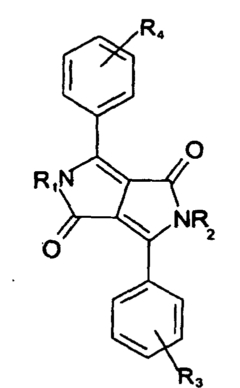 dans laquelle R₁ et R₂ représentent indépendamment un atome d'hydrogène, un radical alkyle en C₁ à C₁₈, alcényle en C₃ à C₁₂, alkoxycarbonyle en C₂ à C₅, phényle éventuellement substitué par un halogène, R₃ et R₄ représentent indépendamment un atome d'hydrogène, un groupement - SO₃M, un radical alkyle en C₁ à C₁₈, alkoxy en C₁ à C₄, halogène, phényle, les radicaux alkyle, alcényle et alkoxy pouvant être linéaires ou ramifiés, M représentant un atome d'hydrogène, un atome de métal ou un groupement ammonium.

2. Composition selon la revendication 1, **caractérisée en ce que** R₁ et R₂ représentent indépendamment un atome d'hydrogène, un radical méthyle ou 4-chlorophényle.

3. Composition selon la revendication 1 ou 2, **caractérisée en ce que** R₃ et R₄ représentent indépendamment un atome d'hydrogène, 4-chloro ou 4-ter-butyle.

4. Composition selon l'une des revendications précédentes, **caractérisée en ce que** le pigment est le pyrrolo (3,4 -c) pyrrole-1,4-dione dont les deux cycles benzéniques sont dihydrogénés en 2, 5 et chlorés en 4.

5. Composition selon l'une des revendications précédentes, **caractérisée en ce que** le pigment est présent à raison de 1 à 25% en poids par rapport au poids de la composition.

6. Composition selon l'une des revendications précédentes, se présentant sous forme d'un produit de maquillage de la peau, des lèvres et/ou des fibres kératiniques d'être humain.

7. Composition selon l'une des revendications précédentes, se présentant sous forme d'un vernis à ongles, mascara, eye-liner ou composition capillaire de type laque, rouge à lèvres, brillant à lèvres, fond de teint, produit anti-cernes, fard à joues ou à paupières, poudre, blush, maquillage du corps.

8. Composition selon l'une des revendications précédentes, comprenant, en outre, au moins une phase grasse choisie parmi les huiles, les cires, les gommes, les corps gras pâteux, et leurs mélanges.

9. Composition selon l'une des revendications précédentes, **caractérisée en ce qu'**elle contient, en outre,'une phase particulaire additionnelle pouvant être présente à raison de 0 à 35 % du poids total de la composition.

10. Composition selon l'une des revendications précédentes, se présentant sous forme anhydre.

11. Utilisation cosmétique de la composition selon l'une des revendications précédentes, pour soigner et/ou maquiller et/ou protéger la peau et/ou les lèvres et/ou les fibres kératiniques.

12. Utilisation de la composition cosmétique selon l'une des revendications 1 à 10 pour la préparation d'un onguent destiné à traiter et/ou protéger la peau et/ou les lèvres.

13. Utilisation dans ou pour la fabrication d'une composition colorée cosmétique d'un agent de coloration de formule (A) telle définie dans les revendications 1 à 4 en vue de protéger la peau et/ou les lèvres et/ou les fibres kératiniques contre les méfaits des radicaux libres et/ou lutter contre les signes cutanés du vieillissement.

14. Procédé de protection cosmétique de la peau et/ou des lèvres et/ou des fibres kératiniques, contre les méfaits des radicaux libres et/ou de lutte contre les signes cutanés du vieillissement photoinduit, consistant à appliquer sur la peau et/ou les lèvres et/ou les fibres kératiniques la composition selon l'une des revendications 1 à 10.

## Patentansprüche

1. Farbige kosmetische Zusammensetzung zur topischen Anwendung, **dadurch gekennzeichnet, dass** sie bezogen auf das Gesamtgewicht der Zusammensetzung 0,01 bis 50 Gew.-% eines Pigments der Formel (A) enthält: 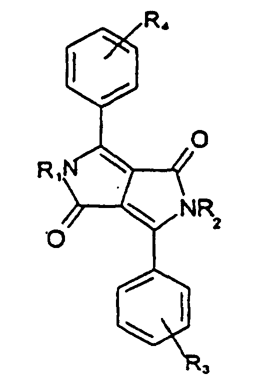 worin bedeuten:
- R₁ und R₂ unabhängig voneinander Wasserstoff, C₁₋₁₈-Alkyl, C₃₋₁₂-Alkenyl, C₂₋₅-Alkoxycarbonyl oder gegebenenfalls mit einem Halogen substituiertes Phenyl und
- R₃ und R₄ unabhängig voneinander Wasserstoff, -SO₃M, C₁₋₁₈-Alkyl, C₁₋₄-Alkoxy, Halogen oder Phenyl, wobei die Alkyl-, Alkenylund Alkoxy-Gruppen geradkettig oder verzweigt vorliegen können und wobei M ein Wasserstoffatom, ein Metallatom oder eine Ammoniumgruppe bedeutet.

2. Zusammensetzung nach Anspruch 1, **dadurch gekennzeichnet, dass** R₁ und R₂ unabhängig voneinander Wasserstoff, Methyl oder 4-Chlorphenyl bedeuten.

3. Zusammensetzung nach Anspruch 1 oder 2, **dadurch gekennzeichnet, dass** R₃ und R₄ unabhängig voneinander Wasserstoff, 4-Chlor oder 4-*tert*-Butyl bedeuten.

4. Zusammensetzung nach einem der vorhergehenden Ansprüche, **dadurch gekennzeichnet, dass** es sich bei dem Pigment um das Pyrrolo[3,4-*c*]pyrrol-1,4-dion handelt, dessen zwei Benzolringe in 2-, 5-Stellung dihydriert und in 4-Stellung chloriert sind.

5. Zusammensetzung nach einem der vorhergehenden Ansprüche, **dadurch gekennzeichnet, dass** das Pigment in einem Anteil von 1 bis 25 Gew.-%, bezogen auf das Gesamtgewicht der Zusammensetzung, vorliegt.

6. Zusammensetzung nach einem der vorhergehenden Ansprüche, die in Form eines Produkts zum Schminken der Haut, der Lippen und/oder der Keratinfasern von Personen vorliegt.

7. Zusammensetzung nach einem der vorhergehenden Ansprüche, die als Nagellack, Mascara, Eyeliner oder als Zusammensetzung für die Haare vom Typ eines Lacks, als Lippenstift, Lippenglanz, Make up, Produkt gegen Augenringe, Rouge, Lidschatten, Puder, Wangenrouge oder als Schminke für den Körper vorliegt.

8. Zusammensetzung nach einem der vorhergehenden Ansprüche, die ferner mindestens eine Fettphase enthält, die unter den Ölen, Wachsen, Gummis, pastösen Fettsubstanzen und deren Gemischen ausgewählt ist.

9. Zusammensetzung nach einem der vorhergehenden Ansprüche, **dadurch gekennzeichnet, dass** sie ferner eine zusätzliche Partikelphase enthält, die in einem Anteil von 0 bis 35 % des Gesamtgewichts der Zusammensetzung vorliegen kann.

10. Zusammensetzung nach einem der vorhergehenden Ansprüche, die in wasserfreier Form vorliegt.

11. Kosmetische Verwendung der Zusammensetzung nach einem der vorhergehenden Ansprüche zur Pflege und/oder zum Schminken und/oder zum Schutz der Haut und/oder der Lippen und/oder der Keratinfasern.

12. Verwendung der kosmetischen Zusammensetzung nach einem der Ansprüche 1 bis 10 zur Herstellung einer Salbe, die zur Behandlung und/oder zum Schutz der Haut und/oder der Lippen bestimmt ist.

13. Verwendung eines wie in den Ansprüchen 1 bis 4 definierten Farbmittels der Formel (A) in einer farbigen kosmetischen Zusammensetzung oder zur Herstellung einer farbigen kosmetischen Zusammensetzung, um die Haut und/oder die Lippen und/oder die Keratinfasern gegen die schädlichen Wirkungen von freien Radikalen zu schützen und/oder die Zeichen der Hautalterung zu bekämpfen.

14. Kosmetisches Verfahren zum Schutz der Haut und/oder der Lippen und/oder der Keratinfasern gegen die schädlichen Wirkungen von freien Radikalen und/oder zur Bekämpfung der Zeichen der lichtinduzierten Hautalterung, das darin besteht, die Zusammensetzung nach einem der Ansprüche 1 bis 10 auf die Haut und/oder die Lippen und/oder die Keratinfasern aufzubringen.

## Claims

1. Coloured cosmetic composition for topical application, **characterized in that** it comprises 0.01 to 50% by weight, with respect to the weight of the composition, of a pigment of formula (A): 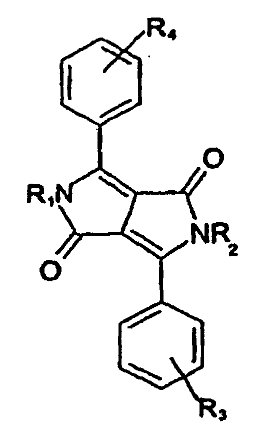 in which R₁ and R₂ independently represent a hydrogen atom, a C₁ to C₁₈ alkyl radical, a C₃ to C₁₂ alkenyl radical, a C₂ to C₅ alkoxycarbonyl radical or a phenyl radical which is optionally substituted by a halogen and R₃ and R₄ independently represent a hydrogen atom, an -SO₃M group, a C₁ to C₁₈ alkyl radical, a C₁ to C₄ alkoxy radical, a halo radical or a phenyl radical, it being possible for the alkyl, alkenyl and alkoxy radicals to be linear or branched and M representing a hydrogen atom, a metal atom or an ammonium group.

2. Composition according to Claim 1, **characterized in that** R₁ and R₂ independently represent a hydrogen atom or a methyl or 4-chlorophenyl radical.

3. Composition according to Claim 1 or 2, **characterized in that** R₃ and R₄ independently represent a hydrogen atom, 4-chloro or 4-tert-butyl.

4. Composition according to one of the preceding claims, **characterized in that** the pigment is pyrrolo[3,4-c]pyrrole-1,4-dione, the two benzene rings of which are dihydrogenated at the 2 and 5 positions and chlorinated at the 4 position.

5. Composition according to one of the preceding claims, **characterized in that** the pigment is present in a proportion of 1 to 25% by weight with respect to the weight of the composition.

6. Composition according to one of the preceding claims, which is provided in the form of a product for making up the human skin, lips and/or keratinous fibres.

7. Composition according to one of the preceding claims, which is provided in the form of a nail varnish, mascara, eyeliner, hair composition of lacquer type, lipstick, lip gloss, foundation, product for concealing rings under the eyes, face powder, eyeshadow, powder, blusher or make-up for the body.

8. Composition according to one of the preceding claims, additionally comprising at least one fatty phase chosen from oils, waxes, gums, pasty fatty substances and their mixtures.

9. Composition according to one of the preceding claims, **characterized in that** it comprises, moreover, an additional particulate phase which can be present in a proportion of 0 to 35% of the total weight of the composition.

10. Composition according to one of the preceding claims, which is provided in anhydrous form.

11. Cosmetic use of the composition according to one of the preceding claims for caring for and/or making up and/or protecting the skin and/or lips and/or keratinous fibres.

12. Use of the cosmetic composition according to one of Claims 1 to 10 for the preparation of a salve intended to treat and/or protect the skin and/or lips.

13. Use, in or for the manufacture of a coloured cosmetic composition, of a colouring agent of formula (**A**) as defined in Claims 1 to 4 for the purpose of protecting the skin and/or lips and/or keratinous fibres against the damaging effects of free radicals and/or combating cutaneous signs of ageing.

14. Process for the cosmetic protection of the skin and/or lips and/or keratinous fibres against the damaging effects of free radicals and/or for combating cutaneous signs of photoinduced ageing, which consists in applying the composition according to one of Claims 1 to 10 to the skin and/or lips and/or keratinous fibres.
